# EUROPEAN PATENT SPECIFICATION

(11) **EP 0 483 514 B1**
(45) Date of publication and mention of the grant of the patent: **02.11.2000**
(21) Application number: 91116407.7
(22) Date of filing: 26.09.1991
(51) Int. Cl.: C12Q 1/68, C07H 21/04

(54) **Use of molecular markers in tree breeding**
Verwendung von molekularmarkern in der Baumzüchtung
Utilisation de marqueurs moléculaires dans l'élevage d'arbres

(30) Priority: 01.10.1990 GB 9021342
(43) Date of publication of application: 06.05.1992
(73) Proprietor: ADVANCED TECHNOLOGIES (CAMBRIDGE) LTD., London WC2R 3LA (GB)
(72) Inventor: Read, Graham Albert, Ampfield, Hampshire (GB); Ward, Martin Richard, Ely, Cambridgeshire (GB)
(74) Representative: MacLean, Kenneth John Hamson

(56) References cited:
- EP-A- 0 221 633
- EP-A- 0 306 139
- WO-A-84/04758
- GENETICS, vol. 123, November 1989, pages 557-565, The Genetics Society of America, Austin, Texas, US; P. KEIM et al.: "Genetic analysis of an interspecific hybrid swarm of populus: occurrence of unidirectional introgression"
- THEOR. APPL. GENET., vol. 76, 1988, pages 841-845, New York, US; A.E. SZMIDT et al.: "Classifying seedlots of Picea sitchensis and P. glauca in zones of introgression using restriction analysis of chloroplast DNA"
- ILLUS. MAPS. PAPER., 0 (0), 1990, pages 244-245, Stuttgart, DE; J.H. CULPEPPER et al.: "Characterization of Cornus species and C. florida cultivars using RFLP", & FAST GROWING TREES AND NITROGEN FIXING TREES; INTERNATIONAL CONFERENCE, Marburg, 8th - 12th October 1989 (Cat. O)
- PLANT BIOLOGY, HORTICULTURAL BIOTECHNOLOGY, vol. 11, 1990, pages 335-346, New York, US; M. WALTON: "Application of RFLP technology to applied plant breeding", & HORTICULTURAL BIOTECHNOLOGY SYMPOSIUM, Davis, CA, 21st - 23rd August 1989 (Cat. O)
- PLANT BIOLOGY, HORTICULTURAL BIOTECHNOLOGY, vol. 11, 1990, pages 247-259, New York, US; J.W. DeVERNA et al.: "RFLP technology"
- CHEMICAL ABSTRACTS, vol. 111, 1989, page 154, abstract no. 226055u, Columbus, Ohio, US; V. PETHE et al.: "Restriction fragment length polymorphism: a recent approach in plant breeding", & INDIAN J. BIOCHEM. BIOPHYS. 1989, 26(5), 285-8
- Isozymes in plant genetics and breeding, Part A, ELSEVIER SCIENCE PUBLISHERS B.V., Amsterdam, NL, Tanksley and Orton (editors), 1983, pp. 381-400, W. T. Adams
- SCHUCH: "Advances in plant biotechnology and their implication for forestry research", IN VITRO CELL. DEV. BIOL., , July 1991, vol. 27, no. , pages 99 to 103

## Description

The potential use of molecular markers for use in plant breeding has been discussed at length (Burr et al 1983; Tanksley 1983; Beckmann and Soller, 1986) and in a number of annual crops such as corn (Helentjaris et al 1986), tomatoes (Bernatzky and Tanksley 1986; Helentjaris et al 1986), lettuce (Landry et al 1987), and rice (McCouch et al 1988) chromosome maps have been constructed using a combination of RFLPs and isozyme loci. These maps and/or associated molecular probes (Patents WO 84/04758; EP 242062; WO 89/07647) have been used to identify and thereby protect inbred lines or varieties; to breed for quantitative or polygenic traits; for selection of specific traits in segregating seedling populations; and to determine phylogenetic relationships within genera and species.

The improvement of woody tree species presents special problems (Zobel and Talbert, 1984), amongst which are poorly characterised germplasm, a restriction to half-sib analysis of progeny trials from random matings in seed orchards and variable seed quality from year to year.

One technique which has been used in an attempt to overcome some of these problems is isozyme analysis. This has been used for determining genetic relatedness of trees and as a means for assessing degree of selfing and hence potential quality of seedlots (Adams 1983). However, this method suffers from the drawback that although differences between isozyme patterns indicates a true difference, similarity in pattern does not necessarily imply overall genetic similarity as unique isozyme markers may not always be present.

One area where RFLP technology has been applied to a forestry problem was the use of restriction fragment analysis of chloroplast DNA to determine the degree of introgression in natural seedlots of geographically overlapping populations of two spruce species (Szmidt 1988). However, this approach is limited by its assessment of only a small part of the genome which may not reflect diversity at or below the species level.

The use of total genomic DNA for the genetic description of natural populations of two tree species and their interspecific hybrids using RFLP technology has also been studied (Keim et al 1989).

An object of the present invention is to provide a method by which trees can be selected based not only on phenotypic characteristics but also on genetic diversity. This allows the tree breeder to construct more efficient crossing programmes and to ensure maximum genetic diversity in their breeding populations. This gives an economic gain based on a greater proportion of vigorous high yielding trees produced from the collected seed.

In clonal forestry plantings this invention can be used to select clones based on their genetic diversity rather than just performance. Lack of diversity in clonal plantings may lead to catastrophic losses from adverse conditions affecting all similar clones to the same extent at the same time, whereas in plantings selected on genetic diversity these risks are reduced as truly different clones may react in different ways to adverse conditions. For example pest or disease resistance may be present in some clones and not in others. Defined levels of diversity, in terms of clone numbers per unit area, is mandatory to clonal planting in some countries of the world. This invention would allow diversity to be measured on real differences rather than on numbers which may have no significance if all selected clones have similar genetic backgrounds.

The present invention is of advantage in the breeding of commercial tree types, for example forest trees (Eucalyptus, Pinus, Picea and Populus for instance).

The present invention provides a method of forest tree breeding wherein Restriction Fragment Length Polymorphism (RFLP) technology is applied to samples of tree material from a plurality of forest trees; the data derived from said RFLP technology is statistically analysed thereby to cluster genetically similar trees of said plurality of trees; two of said trees of genetic diversity are selected based on the statistically analysed RFLP data; and a further tree or trees is/are derived from the two selected trees.

Restriction Fragment Length Polymorphism (RFLP) technology is applied to samples of tree material from a plurality of trees; the information derived from the RFLP technology is presented in a genetic relatedness hierarchy, a level, e.g. the lowest, in said hierarchy comprising groups, each of which groups relates two of the trees as being more genetically related to each other than either of the two trees is genetically related to any other tree in any other of said groups; two of said trees of appropriate relative genetic diversity are selected; and a further tree or trees is/are derived from the two selected trees.

A further tree(s) may be derived by crossing the selected pair of trees. Alternatively, further trees may be derived by cloning each of the two selected trees.

In a practical application of the present invention with a view to deriving progeny by crossing, the RLFP technology is carried out so as to select a multiplicity of pairs of parent trees from which to derive the progeny.

The tree material to which the RFLP technology is applied is suitably leaf or shoot material, preferably young leaf or shoot material. Preferably, the DNA extracted for the application thereto of the RFLP technology is total genomic DNA.

### Description of Figures

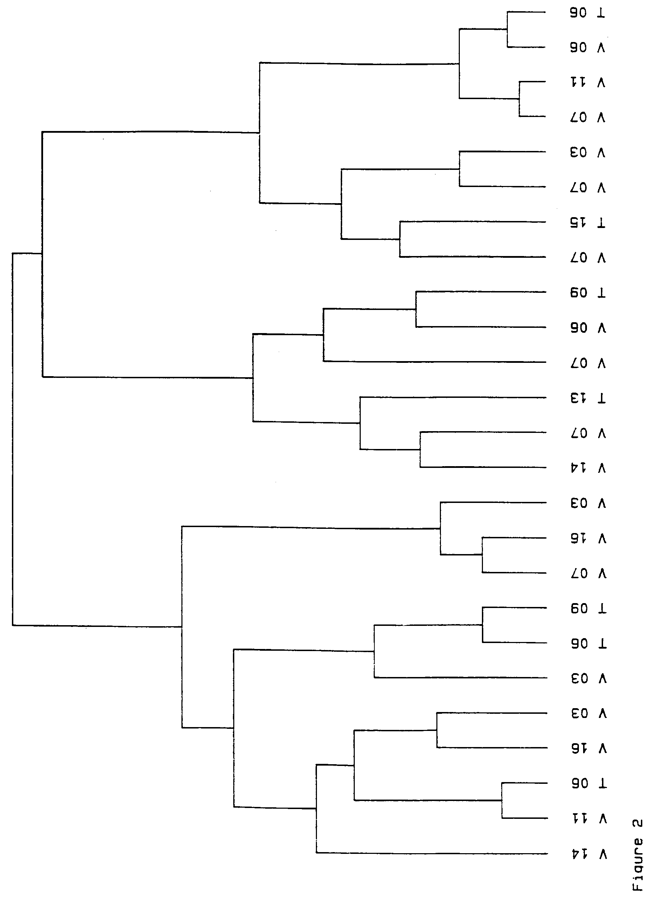

Figure 1 shows a diagrammatic representation of an autoradiogram of DNA from 25 sample trees digested with restriction endonuclease EcoR I and probed with the insert from clone GLPP063. In total 30 discrete fragments are revealed by this probe across these particular trees, but 32 discrete fragements across all trees tested.
   [Table 1 shows the banding pattern data from the autoradiogram of Figure 1, reading down the autoradiogram from larger to smaller sized fragments. The data set for each sample consists of a series of 32 binary digits.]
Figure 2 shows the dendrogram produced by clustering analysis of the data shown in Table 1. Small sub-groups can be seen to be clustering together as a result of a higher degree of similarity between one another than with the other members of the sample population as a whole.
Figure 3 shows the dendrogram produced by clustering analysis of the data produced by several (six) probes. The binary strings are concatenated and read as one large data set (118 digits). Clustering of sub-groups is more defined when using a single probe.
Figure 4 shows the dendrogram produced by clustering analysis of the fingerprint data of six probes with fusion of the last six clusters to produce 6 genetic groups.

### Detailed Description of the preferred method

Genomic DNA Preparation
Genomic Digests and Gel Electrophoresis
Southern Blotting
Probe Libraries
Probe Preparation
Probe Labelling
Hybridisation and Autoradiography
Clone Selection
Band Scoring and Analysis
Diversity Analysis

### Genomic DNA Preparation

DNA preparation is based on a modification of the method of Dellaporta et al (1983). 10g samples of each of the candidate Eucalyptus globulus trees, preferably young leaf tissue, were ground with a pestle and mortar with liquid nitrogen for 3 minutes. 5g Polyvinyl Polypyrollidone were added, followed by 95ml cold Extraction Buffer (50 mM EGTA), 60 mM Magnesium Acetate, 500 mM NaCl, 100 mM Tris-Cl, 20 mM DTT, 50 µg/ml Pronase E; pH 8.0), and then 5 ml 20% SDS was added, the whole being mixed well after each addition. DNA extraction was achieved by incubation at 65°C for 10 minutes. 35ml 5M Potassium Acetate was added and the lysate stood on ice for 30 minutes. Following centrifugation at 11,000g for 10 minutes, the supernatant was filtered through one layer of Miracloth, and 70ml Propan-2-ol added. The mixture was held at -20°C for at least 2 hours and then centrifuged at 12,000*g* for 20 minutes. The DNA pellet was drained well, resuspended in 700 µl 50 mM Tris-Cl/10 mM EDTA; pH 8.0, and transferred to a microfuge tube. Insoluble debris was removed by centrifugation for 2 minutes. 500 µl Propan-2-ol and 70µl 10M Ammonium Acetate was added to the supernatant which was then stood on dry ice for 30 minutes. DNA was pelleted by centrifugation for 10 minutes, drained, and resuspended in 200 µl TE (10 mM Tris-Cl, 1 mM EDTA; pH 8.0).

### Genomic Digests and Gel Electrophoresis

Restriction digestion of genomic DNA was performed with a variety of restriction endonucleases under conditions and in buffers recommended by the suppliers (Life Technologies).

5 µg of each digested DNA sample was loaded on a 0.8% agarose gel in TBE buffer (90 mM Tris, 90 mM Boric Acid, 2 mM EDTA). 400 ng of a Hind III digest of lambda DNA was loaded on the same gel to act as a size standard. Digests were electrophoresed overnight at approximately 1.3 V/cm.

### Southern Blotting

All blots were prepared by capillary transfer by a procedure similar to that of Southern (1975) as described by Sambrook et al (1989a), with the following modifications; (i) the blotting membrane used was Biodyne B (0.45 µm, nylon), (ii) this membrane does not require the gel to be neutralised after the alkaline denaturation step, and, (iii) following transfer, the membrane was baked at 80°C for 30 minutes, and then rinsed in 2xSSC solution (1xSSC is 150 mM NaCl, 15 mM Sodium Acetate; pH 7.2), wrapped in Saran Wrap, and stored at -20°C till required.

### Probe Libraries

All the clones used were derived from two E. globulus genomic libraries. Total genomic DNA from E.globulus was prepared as described above, restricted by Pst I, and cloned into plasmid vector pUC9. The clones were maintained as bacterial glycerol cultures at -80°C, and when required grown upon L-agar containing 100 µg/ml ampicillin.

### Probe Preparation

The probe DNA was prepared by Polymerase Chain Reaction (PCR) amplification of the genomic DNA insert in each plasmid clone. This was achieved by suspension of a bacterial colony containing the clone of choice directly in the PCR reaction buffer.

Amplification of the clone inserts was performed with Tag Polymerase in buffer provided by the supplier (Promega) and supplemented with four deoxynucleotide triphosphates (dATP, dGTP, dCTP, dTTP; 200 µmol each), and two oligonucleotide primers (0.25 µg per reaction), in a reaction volume of 50 µl containing 0.25 units of Tag Polymerase. Incubation was performed with the following programme;
94°C x 90sec, 40°C x 10 sec, 70°C x 3 min; 1 cycle
94°C x 10sec, 40°C x 10 sec, 72°C x 3 min; 9 cycles
94°C x 10sec, 40°C x 10 sec, 72°C x 4 min; 10 cycles
94°C x 10sec, 40°C x 10 sec, 72°C x 5 min; 10 cycles
72°C x 5 min; 1 cycle
The following primers were used during PCR;
- RFLP1: ATTACGCCAAGCTTGGCTGCA
- RFLP2: TTCCCGGGGATCCGTCGA
M13 Sequencing and Reverse Sequencing primers could also be used for PCR with all clones.

Following PCR and gel analysis the clones were purified by phenol/chloroform extraction, followed by ethanol precipitation. It was possible to omit the phenol/chloroform extraction step without deleterious effects.

Following DNA estimation by fluorometry (Hoeffer fluorometer) 1ng of lambda DNA was mixed with 50 ng of each insert to highlight the size standards on the genomic blots.

### Probe Labelling

DNA probes were radiolabelled by the random hexamer method of Feinberg and Vogelstein (1983, 1984) using a Pharmacia Oligonucleotide Labelling Kit. 50 µCi of ³²P-dATP were used to label 50ng of probe DNA. After incubation for 2 hr the reaction was stopped and the probe denatured by adding 200 µl water and boiling for 3 minutes. The probe was then immediately placed on ice and added to the hybridisation solution within 10 minutes.

### Hybridisation and Autoradiography

The genomic blot to be hybridised was pre-washed in 2xSSC, then in 1% Triton X-100, and lastly Prehybridisation Solution (detailed below). All solutions were pre-warmed to 55°C. The membrane was then placed in hybridisation bottles with 50-100ml Prehybridisation Solution (5 x SSC, 50 mM Tris-Cl, 5 mM EDTA, 0.2% BSA, 0.2% Ficoll 400,000, 0.2% Polyvinyl Pyrollidone, 0.1% SDS, 0.1% Sodium Pyrophosphate, 0.5% "Blotto"; pH 7.5) and incubated at 55°C in a rotary oven (Hybaid) for at least 5 hours (usually overnight).

The Prehybridisation Solution was discarded and 20ml of fresh solution added (pre-warmed to 55°C). The denatured probe was then added and the bottle replaced in the hybridisation oven and incubated at 55°C for a time determined by the size of the probes and calculated as being within the range 1-3 x cot_{1/2}.

Following hybridisation the membrane was washed at 55°C in a shaking waterbath sequentially in the following 3 solutions; (i) 1l 2xSSC/0.2%SDS for 1 hr, (ii) 1l 0.5xSSC/0.2% SDS for 1 hr, (iii) 1l 0.2XSSC/0.2%SDS for 30 minutes. The membrane was then wrapped in Saran Wrap and autoradiography performed as described by Sambrook et al (1989b).

Following autoradiography, the membrane was stripped for re-probing by incubating with shaking in the following solutions; (i) 0.4M NaOH at 45°C for 30-45 minutes, (ii) 0.1xSSC/0.1%SDS at room temperature for 15 minutes, (iii) 0.2M Tris-HCL pH7.4 at room temperature for 15 minutes. The membrane was then covered with Saran Wrap and stored at -20°C for re-use.

### Clone Selection

Clones from the E.globulus libraries were hybridised against Southern blots of restricted genomic DNA from a small number of Eucalyptus species, interspecific hybrids, and E.globulus provenances. Those clones which showed the highest degree of discrimination between these samples, either individually or in concert, were selected for use in the subsequent analysis of a wider range of E.globulus provenances.

### Band Scoring and Analysis

On development of the autoradiograms the hybridising fragments (bands) present from each genomic DNA were scored as follows. The position of all bands on each autoradiogram (or each set of autoradiograms when several membranes had been hybridised with the same probe) were located with reference to the lambda DNA size standards. Each genomic DNA was then scored for the presence or absence of each of these bands, the result being recorded as "1" (present) or "0" (absent). The band pattern for each genomic DNA (and thereby each tree) was described by a series of binary digits e.g. the pattern 10010111001 might describe a genomic DNA which exhibited 6 fragments hybridising to a probe which revealed a total of 11 discrete fragment sizes across the whole range of DNAs (trees) screened in this way.

Data obtained from several probes could be combined by appending the binary sequence from each subsequent probe/autoradiogram to produce a longer string of digits. No attempt was made to add weighting to any of the data to reflect different intensities of the bands, or to allow for the fact that different probes revealed the presence of different numbers of bands. When groups of trees were compared, the data from all possible fragments were scored including common bands (present in all cases) and bands absent on individual autoradiograms but found within the group (of trees) on other autoradiograms. This ensured that the fragment pattern of an individual tree was the same irrespective of which other trees were included on the autoradiogram. This ensured that the degree of similarity between any two trees took the same value irrespective of which other trees were also in the analysis.

A "genetic fingerprint" can then be constructed for each tree using the fragment patterns from a number of probes.

### Diversity Analysis

A similarity matrix was computed using similarity coefficients such as Jaccard's Coefficient (Sokal and Sneath, 1963) which uses the number of matching bands between any two samples divided by the total number of matching and unique bands present in the two samples. These coefficients can then be used in a clustering algorithm, for example Unweighted Pair Group Arithmetic Average Clustering Method (UPGMA) or Ward's Method (Wishart, 1987) to group the most similar samples together in dendrograms or other graphical representations.

An example is now given of use of these fingerprints to measure genetic diversity in a commercial tree breeding population.

### EXAMPLE

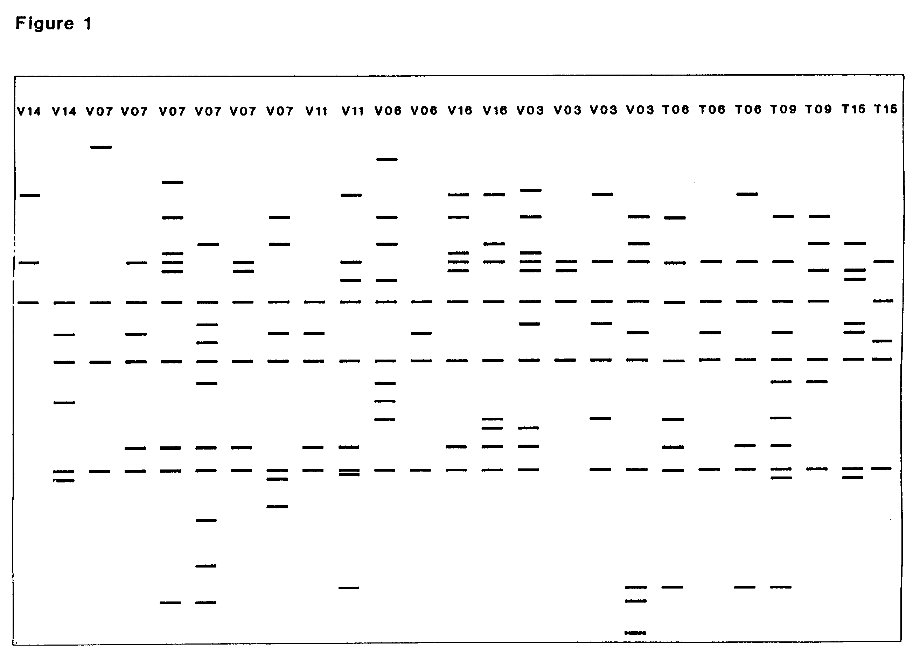

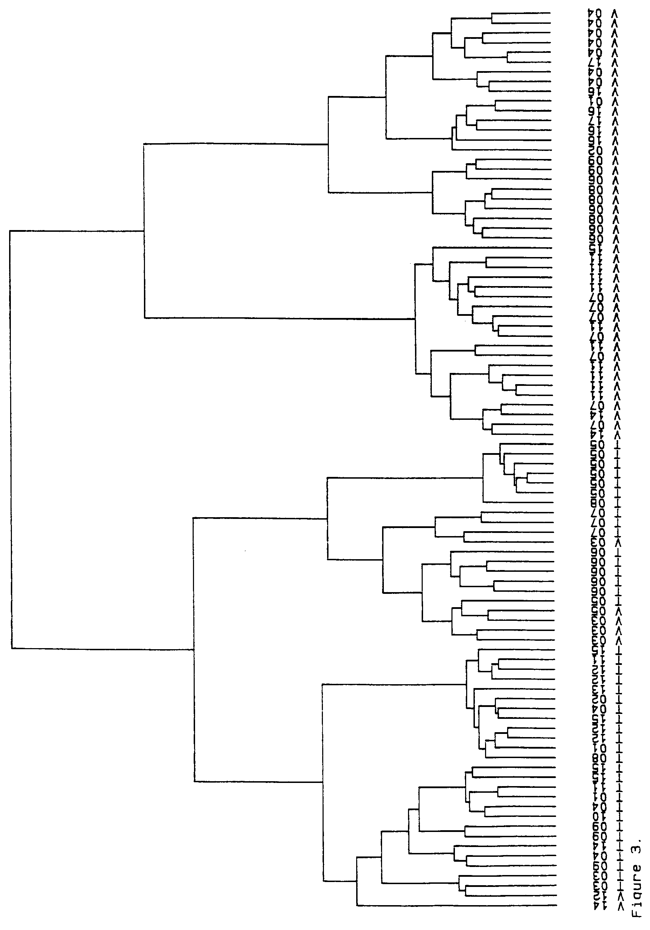

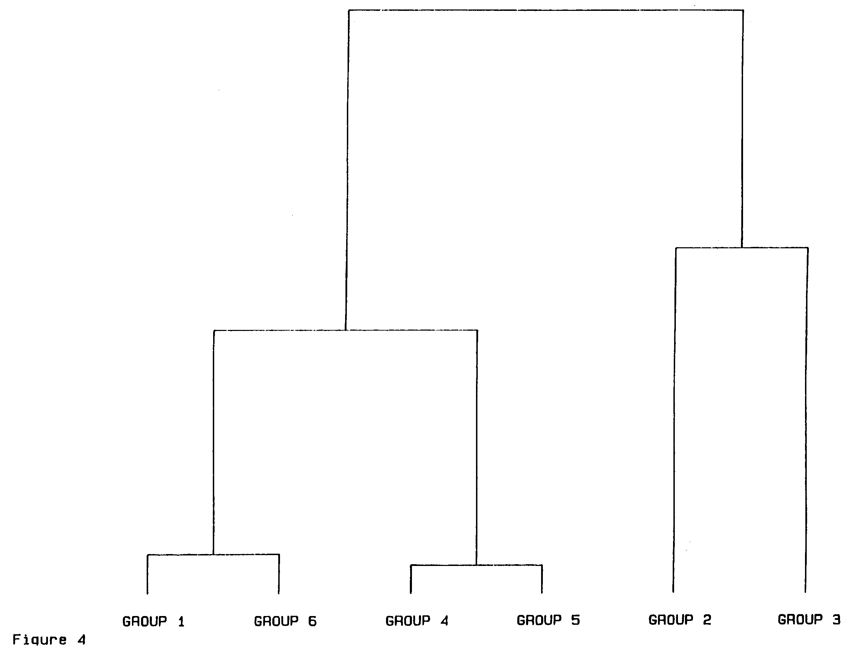

DNA was extracted from 92 randomly selected trees from an E.globulus provenance trial, of known seed origin, in a commercial breeding programme. The origin of this material was from seed collected in 32 locations in the Australian states of Victoria and Tasmania and its nearby islands. Genetic fingerprints were produced with 14 selected probes for each tree as described above. Similarity matrices, using Jaccard's Coefficient of Similarity, were computed and dendrograms produced using Ward's Method for individual probes (an example of the autoradiogram of 25 trees probed with a single probe, GLPP063 and the fingerprint as presence or absence of bands are shown in Figure 1 and Table 1 respectively). The fingerprints from the most discriminatory probes were then combined sequentially and further similarity matrices computed. Once stable groupings were produced (Figure 3), which, in this case was with 6 probes, major groups were assembled (Figure 4). The six probes were GLPP011; GLPP029; GLPP063; GLPP093; P002 and P022. A sample of each of these six probes, harboured in E.Coli, was deposited under the Budapest Treaty on the International Recognition of the Deposit of Micro-organisms for the Purpose of Patent Procedure, at the National Collection of Industrial and Marine Bacteria, Aberdeen, Scotland on 17th September 1991 under the respective accession numbers NCIMB 40444; NCIMB 40443; NCIMB 40442; NCIMB 40441; NCIMB 40446 and NCIMB 40445.

In this example, trees in Groups 2 and 3, as a whole, were more dissimilar, that is to say genetically diverse, compared with trees in Groups 1, 6, 4 and 5 and within this latter grouping, trees in Groups 1 and 6 were genetically more diverse than trees in Groups 4 and 5.

It can be seen from Table 2 that Groups 2 and 3 consist solely of Victorian provenance material and that none of these provenances was split between the 2 groups so that Group 2 comprised of 20 trees from 4 different provenances and Group 3 of 24 trees from 8 provenances. The classification for Groups 1, 6, 4 and 5 was not so clear but overall these 4 groups consisted of the Tasmanian provenances and Groups 1 and 6 formed one set with 25 trees from 12 Tasmanian provenances and Groups 4 and 5 another set with 16 trees from 4 other Tasmanian provenances. Only one Tasmanian provenance (T 08) had trees in these two sets. There were, however, 4 Victorian provenances in these two sets, one of which (V 03), was represented by 4 trees which would strongly suggest an unusual origin for this particular provenance.

The discrimination between the Tasmanian and Victorian provenances was not surprising but the degree of discrimination of provenances within these two geographical regions was surprising. What is more, this type of information on genetic diversity can be used by tree breeders to organise their germplasm and help in the decision of what crosses to make or clones to select. Crosses between trees in Groups 1, 6, 4 and 5 and trees in Groups 2 and 3 would create the widest genetic base and crosses within the same groups would significantly reduce genetic diversity. Likewise, clones selected from across all the different Groups would give a wider genetic diversity of planting material than clones selected from within one Group.

This can also be used to measure genetic diversity in natural populations as a means to either deciding which areas contain the most diverse populations or which particular genotypes should be conserved.

### REFERENCES

Adams, W.T. (1983) Application of isozymes in tree breeding. In: Isozymes in Plant Genetics and Preeding.ed. Tanksley, S.D. and Orton, T.J. Part A, pp.381-400, Elsevier, Amsterdam & New York.
Beckmann, J.S. and Soller, M. (1986) Restriction fragment length polymorphisms in plant genetic improvement. In: Oxford Surveys of Plant Molecular and Cell Biology. ed. Miflin, B.J., Vol 3, pp. 196-250, Oxford University Press.
Bernatzky, R. and Tanksley, S.D. (1986) Towards a saturated linkage map in tomato based on isozymes and random cDNA sequences. Genetics 112, 887-898.
Burr, B., Evola, S.V. and Burr, F.A. (1983) The application of restriction fragment length polymorphism to plant breeding. In: Genetic Engineering. Eds Setlow J. and Hollaender A. Vol 5, pp. 45-59. Plenum Press, New York.
Dellaporta, S.J., Wood, J. & Hicks, J.B. (1983) A plant DNA minipreparation: Version II. Plant Mol. Biol. Rep. 1, 19-21.
Feinberg, A.P. and Vogelstein, B. (1983) A technique for radiolabelling DNA restriction endonuclease fragments to high specific activity. Anal. Biochem. 132, 6-13.
Feinberg, A.P. and Vogelstein, B. (1984) Addendum. A technique for radiolabelling DNA restriction endonuclease fragments to high specific activity. Anal. Biochem. 137, 266-267.
Helentjaris, T., Slocum, M., Wright, S., Schaefer, A. and Nienhuis, J. (1986) Construction of genetic linkage maps in maize and tomato using restriction fragment length polymorphisms. Theor. Appl. Genet. 73, 761-769.
Keim, P., Paige, K.N., Whitham, T.G. and Lark, K.G. (1989) Genetic analysis of an interspecific hybrid swarm of Populus: Occurrence of unidirectional introgression. Genetics 123, 557-565.
Landry, B.S., Kesseli, R., Farrara, B. and Michelmore, R.W. (1987) A genetic map of lettuce (Lactuca sativa L.) with restriction fragment length polymorphisms, isozyme, disease resistance and morphological markers. Genetics 116, 331-337.
McCouch, S.R., Kochert, G., Yu, Z.H., Wang, Z.Y., Khush, G.S., Coffmann, W.R. and Tanksley, S.D. (1988) Molecular mapping of rice chromosomes. Theor. Appl. Genet. 76, 815-829.
Sambrook, J., Fritsch, E.F. & Maniatis, T. (1989a) In: Molecular Cloning 2nd edn. Vol 32, pp. E21-E23, Cold Spring Harbor Laboratory Press
Sambrook, J., Fritsch, E.F. & Maniatis T. (1989b) In: Molecular Cloning 2^{nd} edn. Vol 3, pp. E21-E23, Cold Spring Harbor Laboratory Press
Sokal, R.R. and Sneath, P.H.A. (1963) Principles of Numerical Taxonomy. pp. 359, Freeman W.H. & Co., San Francisco
Southern, E.M. (1975) Deletion of specific sequences among DNA fragments separated by electrophoresis J. Mol. Biol. 98, 503-517.
Szmidt, A.E., El-Kassaby, Y.A., Sigurgeisson, A., Alden, T., Lindgren, D. and Hallgren, J.E. (1988) Classifying seedlots of Picea sitchensis and P.Glauca in zones of introgression using restriction analysis of chloroplast DNA. Theor. Appl. Genet. 76, 841-845.
Tanksley, S.D. (1983) Molecular markers in Plant Breeding. Plant Mol. Biol. Rep. 1, 3-8.
Wishart, D. (1987) Clustan Users Manual. Computing Laboratory, University of St. Andrews, Scotland.
Zobel, B. and Talbert, J. (1984) Applied Forest Tree Improvement. pp. 505, John Wiley & Sons, New York.

**Table 1**

| Fingerprints as presence (1) or absence (0) of bands from one probe GLPP063 for 25 trees from Tasmanian and Victorian provenances. **BAND PATTERN** | |
|---|---|
| V14 | 00000100010010000000000000000000 |
| V14 | 00000000000010101010001010000000 |
| V07 | 10000000000010001000001000000000 |
| V07 | 00000000010010101000011000000000 |
| V07 | 00010010111010001000011000000100 |
| V07 | 00000001000011011100011000110100 |
| V07 | 00000000011010001000011000000000 |
| V07 | 00000011000010101000001011000000 |
| V11 | 00000000000010101000011000000000 |
| V11 | 00000100010110001000011100001000 |
| V06 | 01000011000110001111001000000000 |
| V06 | 00000000000010101000001000000000 |
| V16 | 00000110111010001000011000000000 |
| V16 | 00000101010010001001111000000000 |
| V03 | 00001010111011001000111000000000 |
| V03 | 00000000011010001000000000000000 |
| V03 | 00000100010011001001001000000000 |
| V03 | 00000011010010101000001000001110 |
| T06 | 00000010010010001001011000001000 |
| T06 | 00000000010010101000001000000000 |
| T06 | 00000100010010001000011000001000 |
| T09 | 00000010010010101101011010001000 |
| T09 | 00000011001010001100001000000000 |
| T13 | 00000001001101101000001010000000 |
| T15 | 00000000010010011000001000000000 |

**TABLE2**

| Distribution of trees in 6 Groups for E.globulus Tasmanian provenances (T 01 - T 15) and Victorian provenances (V 01 - V 17). | | | | | |
|---|---|---|---|---|---|
| **GROUP 1** | **GROUP 6** | **GROUP 4** | **GROUP 5** | **GROUP 2** | **GROUP 3** |
| T 01 | T 01 | T 05 | T 05 | V 07 | V 01 |
| T 03 | T 02 | T 06 | T 05 | V 07 | V 02 |
| T 03 | T 04 | T 06 | T 05 | V 07 | V 04 |
| T 04 | T 08 | T 06 | T 05 | V 07 | V 04 |
| T 04 | T 11 | T 06 | T 05 | V 07 | V 04 |
| T 09 | T 12 | T 06 | T 05 | V 07 | V 04 |
| T 09 | T 12 | T 07 | T 08 | V 07 | V 04 |
| T 09 | T 12 | T 07 | | V 11 | V 04 |
| T 10 | T 12 | T 07 | | V 11 | V 04 |
| T 11 | T 13 | V 03 | | V 11 | V 06 |
| T 14 | T 15 | V 03 | | V 11 | V 06 |
| T 15 | T 15 | V 03 | | V 11 | V 06 |
| T 15 | | V 03 | | V 11 | V 06 |
| V 12 | | V 05 | | V 11 | V 08 |
| V 14 | | | | V 11 | V 08 |
| | | | | V 11 | V 08 |
| | | | | V 11 | V 09 |
| | | | | V 14 | V 09 |
| | | | | V 14 | V 16 |
| | | | | V 15 | V 16 |
| | | | | | V 16 |
| | | | | | V 16 |
| | | | | | V 17 |
| | | | | | V 17 |

## Claims

1. A method of forest tree breeding wherein Restriction Fragment Length Polymorphism (RFLP) technology is applied to samples of tree material from a plurality of forest trees; the data derived from said RFLP technology is statistically analysed thereby to cluster genetically similar trees of said plurality of said trees; two of said trees of genetic diversity are selected based on the statistically analysed RFLP data; and a further tree or trees is/are derived from the two selected trees.

2. A method according to Claim 1, wherein a further tree is derived by crossing the two selected trees.

3. A method according to Claim 1, wherein further trees are derived by cloning each of the two trees.

4. A method according to Claim 1, 2 or 3, wherein said trees are of a commercial species.

5. A method according to Claim 4, wherein said species is a Eucalyptus species.

6. A method according to any one of the preceding claims, wherein said samples are leaf samples.

7. A method according to any one of Claims 1 to 5, wherein said samples are shoot samples.

8. A method according to any one of the preceding claims, wherein the probes used in the RFLP technology comprise one or more of the subjects of deposits NCIMB 40444; NCIMB 40443; NCIMB 40442; NCIMB 40441; NCIMB 40446 and NCIMB 40445.

## Patentansprüche

1. Verfahren zur Züchtung von Waldbäumen, bei dem die Technik des Restriktionsfragmentlängen-Polymorphismus (RFLP-Technik) auf Proben aus Baummaterial aus einer Vielzahl von Waldbäumen angewandt wird, die Daten, die aus dieser RFLP-Technik abgeleitet werden, statistisch analysiert werden, um so genetisch ähnliche Bäume aus der Vielzahl von Bäumen zusammen zu fassen (Cluster), auf Basis der statistisch analysierten RFLP-Daten zwei der Bäume mit genetischer Diversität selektiert werden; und ein weiterer Baum oder Bäume aus diesen zwei selektierten Bäumen abgeleitet wird bzw. werden.

2. Verfahren gemäß Anspruch 1, worin ein weiterer Baum durch Kreuzen der zwei selektierten Bäume abgeleitet wird.

3. Verfahren gemäß Anspruch 1, worin weitere Bäume durch Klonieren eines jeden der zwei Bäume abgeleitet werden.

4. Verfahren gemäß Anspruch 1, 2 oder 3, worin die Bäume einer handelsüblichen Spezies angehören.

5. Verfahren gemäß Anspruch 4, worin es sich bei der Spezies um eine Eukalyptus-Spezies handelt.

6. Verfahren gemäß einem der vorstehenden Ansprüche, worin es sich bei den Proben um Blattproben handelt.

7. Verfahren gemäß einem der Ansprüche 1 bis 5, worin es sich bei den Proben um Sprossproben handelt.

8. Verfahren gemäß einem der vorstehenden Ansprüche, wobei die Proben, die in der RFLP-Technik eingesetzt werden, einen oder mehrere der Gegenstände der Hinterlegungen NCIMB 40 444, NCIMB 40 443, NCIMB 40 442, NCIMB 40 441, NCIMB 40 446 und NCIMB 40 445 umfassen.

## Revendications

1. Procédé de sélection d'arbres forestiers, dans lequel la technologie du Polymorphisme de Taille des Fragments de Restriction (PTFR) est appliquée à des échantillons de matériel d'arbres provenant d'une pluralité d'arbres forestiers ; les données obtenues par cette technologie PTFR font l'objet d'une analyse statistique, de façon à regrouper des arbres génétiquement similaires de ladite pluralité d'arbres ; deux desdits arbres ayant une diversité génétique sont sélectionnés sur la base des données de la PTFR ayant subi l'analyse statistique ; et un ou plusieurs autres arbres sont obtenus à partir des deux arbres sélectionnés.

2. Procédé selon la revendication 1, dans lequel un autre arbre est obtenu par croisement des deux arbres sélectionnés.

3. Procédé selon la revendication 1, dans lequel d'autres arbres sont obtenus par clonage de chacun des deux arbres.

4. Procédé selon la revendication 1, 2 ou 3, dans lequel lesdits arbres sont d'une espèce commerciale.

5. Procédé selon la revendication 4, dans lequel ladite espèce est une espèce d'Eucalyptus.

6. Procédé selon l'une quelconque des revendications précédentes, dans lequel lesdits échantillons sont des échantillons de feuilles.

7. Procédé selon l'une quelconque des revendications 1 à 5, dans lequel lesdits échantillons sont des échantillons de pousses.

8. Procédé selon l'une quelconque des revendications précédentes, dans lequel les sondes utilisées dans la technologie PTFR comprennent un ou plusieurs des sujets des dépôts NCIMB 40444, NCIMB 40443, NCIMB 40442, NCIMB 40441, NCIMB 40446 et NCIMB 40445.
